# EUROPEAN PATENT APPLICATION

(11) **EP 1 978 099 A1**
(43) Date of publication of application: **08.10.2008**
(21) Application number: 05805421.4
(22) Date of filing: 04.11.2005
(51) Int. Cl.: C12P 1/04, C07G 11/00

(54) **ANTIBACTERIAL SUBSTANCE DM0507 AND UTILIZATION OF THE SAME**

(71) Applicant: AIM Asset Management Co., Ltd., Tokyo 102-0073 (JP)
(72) Inventor: YAMASHITA, Naomi, Maebashi-shi Gunma 371-0831 (JP); IIZUKA, Takeru, Maebashi-shi Gunma 371-0831 (JP); NAKAE, Taiji, Isehara-shi Kanagawa 259-1113 (JP); SATAKE, Sachiko, Takasaki-shi Gunma 370-0841 (JP)
(74) Representative: Hartz, Nikolai
(86) International application number: PCT/JP2005/020318
(87) International publication number: WO 2007/052356

(57) **Abstract**

An object is to provide a more potent novel antimicrobial active substance, which is derived from a naturally occurring substance and has a broad antimicrobial spectrum, and an antimicrobial active substance DM0507 obtained by a production process comprising the following steps (a) to (d):
(a) culturing Bacillus subtilis;
(b) collecting the supernatant from the obtained culture;
(c) collecting the precipitate formed by adjusting the pH of the supernatant to 3 or lower; and
(d) performing extraction from the precipitate with ethanol is provided.

## Description

### Technical Field

The present invention relates to a novel antimicrobial active substance, and more particularly, it relates to an antimicrobial active substance DM0507 having an antimicrobial activity against a broad spectrum of strains and the use thereof.

### Background Art

It is known that microorganisms produce an antimicrobial active substance having such as an antibacterial, antifungal or antiviral activity, and penicillin produced by strains of the genus Penicillium, cephalosporin produced by the genus Cephalosporium, streptomycin, tetracycline, erythromycin produced by the genus Streptomyces and the like have been widely used as antimicrobial active substances.

It is also known that the use of these antimicrobial active substances for many years results in the occurrence of bacteria having resistance to these antimicrobial active substances (resistant bacteria), therefore there has been a demand for a more potent novel antimicrobial active substance.

### Disclosure of the invention

### Problems that the Invention is to Solve

Accordingly, an object of the present invention is to provide a more potent novel antimicrobial active substance which is derived from a naturally occurring substance and has a broad antimicrobial spectrum.

### Means for Solving the Problems

The present inventors have made extensive studies in order to accomplish the above-mentioned object, and as a result, they found that a substance having an antimicrobial activity against a broad spectrum of strains can be obtained from a culture medium of a special strain of Bacillus subtilis, thus the present invention has been completed. In addition, they found that the above-mentioned substance having an antimicrobial activity can exhibit the antimicrobial activity even if it is added to any of a variety of existing products, thus the present invention has been completed.

That is, the present invention is directed to an antimicrobial active substance DM0507, characterized by obtaining by a production process comprising the following steps (a) to (d):
(a) culturing Bacillus subtilis;
(b) collecting the supernatant from the obtained culture;
(c) collecting the precipitate formed by adjusting the pH of the supernatant to 3 or lower; and
(d) performing extraction from the precipitate with ethanol.

Further, the present invention is directed to an antimicrobial agent containing the above-mentioned antimicrobial active substance DM0507 as an active ingredient.

Further, the present invention is directed to a food or drink containing the above-mentioned antimicrobial active substance DM0507.

Further, the present invention is directed to a process for producing an antimicrobial active substance DM0507, characterized by comprising the following steps (a) to (d):
(a) culturing Bacillus subtilis;
(b) collecting the supernatant from the obtained culture;
(c) collecting the precipitate formed by adjusting the pH of the supernatant to 3 or lower; and
(d) performing extraction from the precipitate with ethanol.

### Advantage of the Invention

The antimicrobial active substance DM0507 of the present invention has an antimicrobial activity against a broad spectrum of strains.

Accordingly, by using the antimicrobial active substance DM0507 of the present invention, a variety of antimicrobial agents, foods, drinks, etc. having an excellent antimicrobial activity can be obtained.

### Best Mode for Carrying Out the Invention

An antimicrobial active substance DM0507 (hereinafter referred to as simply "DM0507") of the present invention can be obtained by a production process comprising the steps described below:
(a) culturing Bacillus subtilis;
(b) centrifuging the obtained culture and collecting the supernatant;
(c) collecting the precipitate formed by adjusting the pH of the supernatant to 3 or lower; and
(d) performing extraction from the precipitate with ethanol.

The Bacillus subtilis to be used in the culture in the step (a) of the above-mentioned production process is not particularly limited as long as it produces DM0507, however, preferred examples include Bacillus subtilis DB9011 strain (FERM BP-3418) and Bacillus subtilis MBI 600 strain (Becker Underwood, Inc.: SUBTILEX (trade name)), and particularly preferred examples include Bacillus subtilis DB9011 strain.

Since the culture of the above-mentioned Bacillus subtilis can be carried out by a standard method, the culture conditions are not particularly limited, however, for example, the conditions in which a shaking culture is carried out at 30°C to 40°C, preferably at 35°C for 24 to 48 hours, preferably for 36 hours with Mueller- Hinton broth (manufactured by DIFCO) can be mentioned.

Then, the supernatant is collected from the culture obtained in the above-mentioned step (a) (step (b)). In this step (b), the collection of the supernatant may be carried out by centrifugation. Specific conditions of the centrifugation are not particularly limited, however, for example, the centrifugation may be carried out at 10,000 rpm to 40,000 rpm, preferably at 20,000 rpm for 20 minutes to 1 hour, preferably for 1 hour.

The pH of the supernatant collected in the above-mentioned step (b) is adjusted to 3 or lower, preferably 3.0, and the formed precipitate is collected (step (c)). In this step (c), the adjustment of the pH can be carried out with an acid such as sulfuric acid or hydrochloric acid. Further, after the adjustment of the pH, the supernatant is preferably let stand for 8 to 24 hours, preferably for 12 to 18 hours at 4 to 10°C, preferably at 4 to 5°C. Further, as for the collection of the precipitate formed by adjusting the pH, the collection may be carried out by centrifugation, ultrafiltration, lyophilization, dialysis or the like, preferably centrifugation may be carried out. Specific conditions of the centrifugation are not particularly limited, however, for example, the centrifugation may be carried out at 10,000 rpm to 40,000 rpm, preferably at 20,000 rpm for 20 minutes to 1 hour, preferably for 1 hour.

Lastly, the precipitate collected in the above-mentioned step (c) is subjected to extraction with ethanol (step (d)). By this step (d), DM0507 of the present invention can be obtained. As the method of extracting DM0507 from the precipitate, a method in which ethanol is added to the precipitate, the mixture in a state where the precipitate floats therein is let stand for about 15 minutes to 1 hour, and DM0507 is extracted in ethanol and other methods can be mentioned.

The thus obtained DM0507 of the present invention has a broad spectrum of antimicrobial activity against the following microorganism Groups (A) to (D):
Group (A) (aerobic/ facultative anaerobic microorganisms)
   Streptococcus suis,
   Streptococcus pneumoniae,
   Streptococcus pyogenes,
   Bacillus subtilis ATCC 6633,
   Bacillus cereus var. mycoides ATCC 11778,
   Arcanobacterium pyogenes,
   Aeromonas salmonicida,
   Aeromonas hydrophila,
   Actinobacillus actinomycetemcomitans JCM 2434,
   Actinobacillus pleuropneumoniae type 2,
   Haemophilus parasuis,
   Pasteurella multocida type D,
   Pasteurella haemolytica,
   Salmonella typhimurium,
   Salmonella choleraesuis,
   Klebsiella pneumoniae ATCC 10031,
   Acinetobacter junii,
   Acinetobacter sp.,
   Stenotrophomonas maltophilia,
   Legionella pneumophila serotype 1,
   Mycoplasma gallisepticum, and
   Mycoplasma synoviae;
Group (B) (microaerophilic microorganisms)
   Campylobacter jejuni,
   Campylobacter coli, and
   Helicobacter pylori ATCC 43526;
   Group (C) (obligate anaerobic microorganisms)
   Clostridium perfringens,
   Clostridium difficile,
   Propionibacterium acnes JCM 6425,
   Bacteroides fragilis,
   Porphyromonas gingivalis JCM 8525,
   Tannerella forsythensis JCM 10827,
   Lactobacillus acidophilus JCM 1132,
   Bifidobacterium bifidum JCM 1255,
   Anaerococcus prevotii JCM 6508, and
   Fusobacterium nucleatum; and
Group (D) (fungi)
   Fusarium oxysporum.

DM0507 of the present invention exhibits a high antimicrobial activity particularly against Legionella pneumophila serotype 1, Porphyromonas gingivalis and Propionibacterium acnes among these microorganisms. Next to them, it exhibits a high antimicrobial activity against Clostridium difficile, Campylobacter jejuni, Campylobacter coli and Stenotrophomonas maltophilia, and next to them, Aeromonas salmonicida, Mycoplasma gallisepticum and Mycoplasma synoviae.

Since DM0507 of the present invention has an antimicrobial activity against a broad spectrum of microorganisms as described above, it can be used for an antimicrobial agent containing the substance as an active ingredient, an antimicrobial product, a food or drink, etc. containing the substance.

For the production of the antimicrobial agent containing DM0507 of the present invention as an active ingredient, DM0507 and an appropriate known pharmaceutically acceptable medicinal carrier may be combined and formulated into a preparation by a standard method. The content of DM0507 in this antimicrobial agent is not particularly limited as long as it is an amount capable of exhibiting the antimicrobial activity. However, for example, the MIC (minimum inhibitory concentration) of DM0507 against Acinetobacter junii is measured, and a product may contain DM0507 at the MIC or higher concentrations, preferably 2- to 10-fold the MIC. Hereinafter, a method of measuring the MIC against Acinetobacter junii by a broth microdilution method is shown.

### <Method of measuring MIC against Acinetobacter junii by broth microdilution method>

The measurement was carried out in two series for each sample, and the concentration levels of DM0507 are set up to 10240-fold dilutions by serially diluting a 10-fold dilution 2-fold. First, 100 µl of cation-adjusted Mueller-Hinton broth (hereinafter referred to as CAMHB) is dispensed into each well of a 96-well flat-bottomed microplate. Into the first well to which DM0507 is added, 180 µl of CAMHB is dispensed. Then, 20 µl of DM0507 is added to the first well to make a 10-fold dilution, which is serially diluted 2-fold with a 100-µl aliquot thereof up to 10240-fold. Isolation culture of Acinetobacter junii is carried out on the previous day and culture is carried out in advance with MHA for 18 to 24 hours. The freshly cultured cells on an agar medium is adjusted to a 0.5 McFarland standard with physiological saline and is further diluted 10-fold, and the cell density is adjusted to 10⁷ cfu/ml. Five microliters of the adjusted bacterial suspension is inoculated into each well to give a final inoculum density of 5 x 10⁵ cfu/ml (5 x 10⁴ cfu/100 µl). As controls, a well containing only CAMHB and the bacterial suspension and a well containing only CAMHB are prepared. The microplate is incubated at 35°C for 18 hours, and the MIC is determined to be the lowest dilution that completely inhibited the growth by visual observation.

Further, by incorporating or impregnating DM0507 of the present invention directly or as an antimicrobial agent produced as described above in the existing products shown below, antimicrobial products as shown below can be produced.

### <Quasi-drugs, reagents, gargles, etc.>

Drugs for external use, dentifrices (toothpastes, powder dentifrices and liquid dentifrices), interdental brushes, gargles, disinfectants for such as mouth inflammation, denture products (for washing, attachment, sterilization, disinfection and deodorization), bath agents, medical soaps, lip protectors, nail protectors, outer skin disinfectants, agents for corns or calluses, disinfectants for wounds, protectors for sore or prickly heat, agents for chapped skin, agents for skin dryness or flaking, agents for the scalp, agents for dandruff or itching, and underarm deodorants.

### <Commodities, toiletries, fiber or leather goods, etc.>

Diapers, kitchen goods, toiletries, products obtained by impregnation into soap, carbon fiber, cloth, leather or the like.

### <Washing agents, etc.>

Sanitary cottons (including paper products), shampoos, rinses, sanitary goods and washing agents for contact lens.

### <Cosmetics, etc.>

Cosmetic lotions, emulsions, cleansing agents, esthetic agents, face masks, creams, hand creams, oils for cosmetics, shaving agents and sunscreens.

### <Disinfectants, microbicides, etc.>

Microbicides for circulation type public baths or hot springs, Microbicides for swimming pools, agents for chillers (cooling water) and agents for filters in such as air conditioners.

### <Building materials, coating materials, etc.>

Wood materials, papers, oil paints, water-thinned paints, cloth, adhesives, tatami and protectants for coating materials.

### <Food additives, etc.>

### Preservatives (including drinks)

Among these antimicrobial products, particularly cosmetic lotions and dentifrices are preferred. The production of these antimicrobial products may follow the production process for a common product except for adding an effective amount of DM0507 of the present invention to raw materials of the products.

Further, DM0507 of the present invention can be incorporated in foods and drinks such as sweet stuffs including gums, candies, cookies, tablet-type sweet stuffs and the like, supplements and refreshing drinks. Among these foods and drinks, particularly gums and candies are preferred. By the intake of DM0507 of the present invention on a daily basis by incorporating DM0507 in such a common food or drink, it can be a health food that eliminates bacteria leading to a periodontal disease, Helicobacter pylori or the like, or inhibits the colonization of such bacteria. The production process for these foods and drinks may follow the production process for a common food or drink except for adding an effective amount of DM0507 of the present invention to raw materials of the foods and drinks.

### Examples

Hereinafter, the present invention will be described by way of Examples, however, the present invention is not limited to these Examples.

### Reference Example 1

Screening of bacteria producing antimicrobial active substance:
The antimicrobial activities of the culture supernatants of the following Bacillus subtilis strains were measured by using the growth inhibitory activity against Porphyromonas gingivalis JCM 8525 as an indicator.

### <Test strains>

DB9011 strain (deposited on May 21, 1991 as FERM BP-3418 in International Patent Organism Depositary, National Institute of Advanced Industrial Science and Technology (Central 6, 1-1, Higashi 1-chome, Tsukuba-shi, Ibaraki-ken, 305-8566, Japan)
ATCC 6633 strain
BN1001 strain (manufactured by Meiji Seika Co. : BN-Clean (trade name))
Kubota strain (manufactured by Asahi Biotech KK: BSK strain)
OUV23481 strain (manufactured by Mizkan Group Co.: Kinnotsubu Honegenki (trade name))
MBI 600 strain (manufactured by Becker Underwood, Inc.: SUBTILEX (trade name))

First, each Bacillus subtilis strain was subcultured for three passages on Mueller-Hintons agar medium (manufactured by Eiken Chemical Co., Ltd.). Then, one platinum loop of each strain was inoculated into 10 ml of sterile Mueller-Hinton broth (manufactured by DIFCO), and shaking culture was carried out at 35°C for 24 hours.

Each culture medium after the shaking culture was dispensed into a 1.5 ml microtube, respectively, and centrifugation was carried out at 15,000 rpm for 10 minutes. Then, the centrifuged supernatant was collected, and filtered through a 0.45 µm filter. Then, this filtered culture supernatant was impregnated in a paper disk with a diameter of 6.5 mm (manufactured by DIFCO). On the contrary, Porphyromonas gingivalis to be used as the indicator for antimicrobial activity was anaerobically cultured in GAM bouillon liquid (manufactured by Nissui Pharmaceutical Co., Ltd.) in advance. Then, GAM agar medium (manufactured by Nissui Pharmaceutical Co., Ltd.) was autoclaved and incubated at 50°C. Then, the cultured medium of Porphyromonas gingivalis anaerobically cultured in advance was mixed therewith, which was pored into a dish and allowed to solidify. The paper disk impregnated with each of the filtered culture media of Bacillus subtilis strains was placed on the center of the dish, and anaerobic culture was carried out at 35°C for 48 hours. The diameter of inhibitory zone of each Bacillus subtilis strain for Porphyromonas gingivalis was measured at 48 hours after the initiation of the culture. The results are shown in Table 1.

**[Table 1]**

| Test strain | DB9011 strain | 6633 strain | BN1001 strain | Kubota strain | OUV23481 strain | MBI 600 strain |
|---|---|---|---|---|---|---|
| Inhibitory zone diameter (mm) | 22 | 10 | 9 | 9 | 9 | 22 |

From Table 1, it was found that the culture supernatants of DB9011 strain and MBI 600 strain exhibit a high antimicrobial activity against Porphyromonas gingivalis.

### Example 1

### Production of antimicrobial active substance DM0507:

### (1) Culture of DB9011 strain

DB9011 strain grown on a slant medium and stored in a refrigerator was subcultured on Mueller-Hintons agar medium (manufactured by Eiken Chemical Co., Ltd.), and cultured at 35°C for 24 hours to form a colony. Then, the above-mentioned one colony was inoculated into 10 ml of Mueller-Hinton broth (manufactured by DIFCO) and shaking culture was carried out at 35°C for 24 hours. Finally, 1 ml of the above culture medium was inoculated into 3 L of Mueller-Hinton broth autoclaved in advance, and shaking culture was carried out at 35°C for 30 hours at 200 rpm, whereby a culture medium was obtained.

### (2) Extraction of antimicrobial active substance DM0507

Three liters of the culture medium prepared in the above (1) was centrifuged at 20,000 rpm for 30 minutes while the temperature was maintained at 4°C, and the centrifuged supernatant was collected. Then, the centrifuged supernatant was adjusted to a pH of 3 by adding 10 N hydrochloric acid thereto, and let stand overnight in a refrigerator. Thereafter, the supernatant was further centrifuged at 20,000 rpm for 30 minutes while the temperature was maintained at 4°C, and the supernatant was removed, and then the precipitate was collected. To the precipitate, 10 ml of ethanol (99.5% by mass) was added to allow the precipitate to flow, and the mixture was let stand for about 15 minutes to extract the antimicrobial active substance DM0507. Then, for obtaining this antimicrobial active substance DM0507, centrifugation was further carried out at 20,000 rpm for 30 minutes while the temperature was maintained at 4°C, the supernatant was adjusted to a pH of 7.0 by adding 1 N sodium hydroxide thereto, filtration was carried out with a 0.45 µm filter, and the filtrate was cryopreserved at -80°C.

### (3) Measurement of physiological properties of antimicrobial active substance DM0507

### <IR measurement>

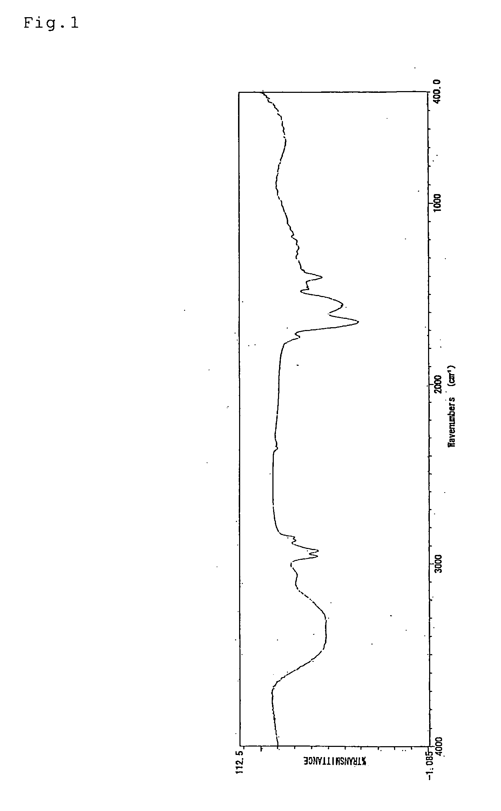

As a result of performing IR measurement of DM0507 under the following conditions, peaks were observed at 3060.48, 2958.27, 2927.41, 2871.49, 2856.06, 1737.55, 1650.77, 1573.63, 1558.20, 1469.49, 1403.92, 1274.72, etc. (all the units are cm⁻¹) (Fig. 1).

### (Measurement conditions)

Measurement apparatus: HORIBA FT-710 (manufactured by Horiba Ltd.)
Measurement method: KBr method
Measurement temperature: room temperature

### <NMR measurement>

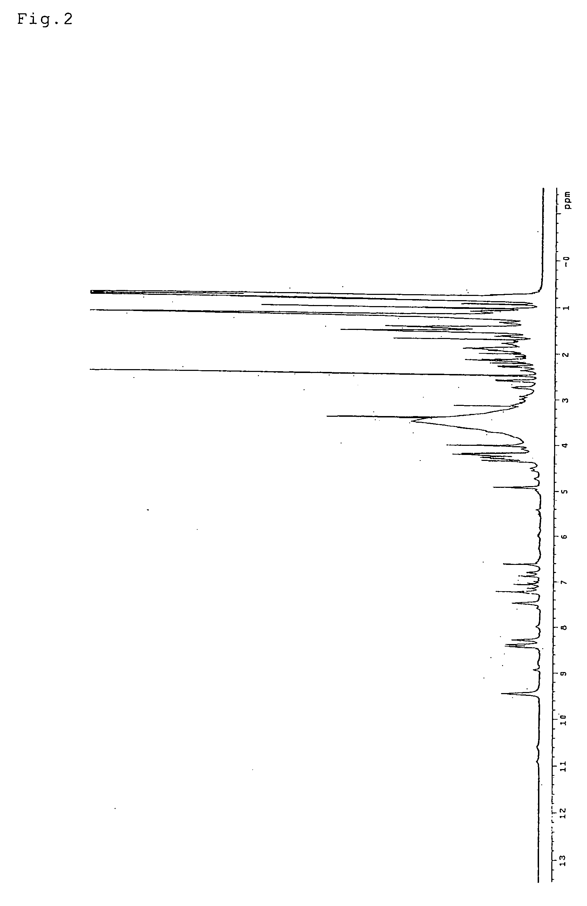

NMR measurement of DM0507 was carried out under the following conditions. These results are shown in Fig. 2.

### (Measurement conditions)

Measurement apparatus: Unity INOVA 600 (manufactured by Varian)

Measurement frequency: 599.898 MHz
Measurement solvent: DMSO-d6
Measurement temperature: 30°C

### Example 2

### Measurement of antimicrobial activity

By using the antimicrobial active substance DM0507 obtained in Example 1, the antimicrobial activity against the following test strains were measured.

### <Test strains>

Staphylococcus aureus MSSA,
Staphylococcus aureus MRSA,
Staphylococcus hyicus,
Micrococcus luteus ATCC 9341,
Streptococcus mutans JCM 5705,
Streptococcus suis,
Streptococcus pneumoniae,
Streptococcus pyogenes,
Enterococcus gallinarum,
Bacillus subtilis DB9011,
Bacillus subtilis ATCC 6633,
Bacillus cereus var. mycoides ATCC 11778,
Arcanobacterium pyogenes,
Vibrio parahaemolyticus ATCC 17802,
Aeromonas salmonicida,
Aeromonas hydrophila,
Actinobacillus actinomycetemcomitans JCM 2434,
Actinobacillus pleuropneumoniae type 2,
Haemophilus parasuis,
Pasteurella multocida type D,
Pasteurella haemolytica,
Pasteurella trehalosi,
Escherichia coli,
Salmonella typhimurium,
Salmonella choleraesuis,
Klebsiella pneumoniae ATCC 10031,
Pseudomonas aeruginosa,
Pseudomonas aeruginosa pump-hyperexpressing strain,
Pseudomonas aeruginosa pump deficient strain,
Acinetobacter junii,
Acinetobacter sp.,
Stenotrophomonas maltophilia,
Legionella pneumophila serotype 1,
Mycoplasma gallisepticum,
Mycoplasma synoviae,
Campylobacter jejuni,
Campylobacter coli,
Helicobacter pylori ATCC 43526,
Clostridium perfringens,
Clostridium difficile,
Propionibacterium acnes JCM 6425,
Bacteroides fragilis,
Porphyromonas gingivalis JCM 8525,
Tannerella forsythensis JCM 10827,
Lactobacillus acidophilus JCM 1132,
Bifidobacterium bifidum JCM 1255,
Candida albicans,
Cryptococcus sp.,
Aspergillus niger, and
Fusarium oxysporum

### (1) Assay

The test strains were subcultured for three passages with an appropriate medium under appropriate culture conditions (Table 2), respectively, and inoculated into the medium by the (2) pour method or the (3) streak method described below. Then, 20 µl of the antimicrobial active substance 0507 prepared in Example 1 was impregnated in respective thin-model paper disks with a diameter of 8 mm (manufactured by Advantech), and ethanol was evaporated by letting the dishes stand in a refrigerator for 30 minutes in a state that the dish covers were opened slightly. As a control, the same procedure was carried out for a disk impregnated only with ethanol. One piece of the DM0507 disk and the control disk was placed on the medium into which any of the test strains was inoculated and incubation was carried out. Then, the inhibitory zone around the disk was measured.

### (2) Pour method

The strain subcultured for three passages was adjusted to a 2 McFarland standard (6.0 x 10⁸/ml) with 1 ml of sterile physiological saline and is further diluted 10-fold. Then, this bacterial suspension was added and mixed with an agar medium which had been autoclaved in advance and incubated at 50°C at a ratio of 0.1% by mass. Further, 10 ml of the medium containing the bacterial suspension was overlayered onto a dish into which 10 ml of an agar medium had been poured in advance. At this time, the cell number was 1.0 x 10⁴/cm².

### (3) Streak method

The strain subcultured for three passages was adjusted to a 0.5 McFarland standard (1.5 × 10⁸/ml) with 1 ml of sterile physiological saline, which was impregnated in a sterile cotton swab on a wood stick (manufactured by Eiken Kizai Co.,Ltd), and streaking was carried out in three directions on each medium.

**[Table 2-1]**

| Strain name | Inhibitory zone diameter (mm) | Assay method | Agar medium 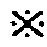 1 | Culture method | Culture temperature (°C) | Culture time (h) |
|---|---|---|---|---|---|---|
| S. aureus MSSA | ≤8 | Pour | MHA | Aerobic | 35 | 24 |
| S. aureus MRSA | ≤8 | Pour | MHA | Aerobic | 35 | 24 |
| S. hyicus | ≤8 | Pour | MHA | Aerobic | 35 | 24 |
| M. luteus ATCC 9341 | ≤8 | Pour | MHA | Aerobic | 35 | 24 |
| S. mutans JCM 5705 | ≤8 | Pour | GAM | CO₂ | 35 | 24 |
| S. suis | 17 | Pour | GAM | CO₂ | 35 | 24 |
| S. pneumoniae | 14 | Streak | MHA supplemeted with sheep blood | CO₂ | 35 | 24 |
| S. pyogenes | 14 | Streak | MHA supplemented with sheep blood | CO₂ | 35 | 24 |
| E. gallinarum | ≤8 | Pour | GAM | CO₂ | 35 | 24 |
| B. subtilis DB9011 strain | ≤8 | Pour | MHA | Aerobic | 35 | 24 |
| B. subtilis ATCC 6633 | 15 | Pour | MHA | Aerobic | 35 | 24 |
| B. cereus ATCC 11778 | 13 | Pour | MHA | Aerobic | 35 | 24 |
| A. pyogenes | 20 | Pour | Sawada | CO₂ | 35 | 24 |
| V. parahaemolyticus ATCC 17802 | ≤8 | Pour | MHA supplemente d with 3% NaCl | Aerobic | 35 | 24 |
| A. salmonicida | 22 | Pour | MHA | Aerobic | 25 | 24 |
| A. hydrophila | 13 | Streak | MHA | Aerobic | 35 | 24 |
| A. actinomycetemcomitans JCM 2434 | 10 | Pour | GAM | CO₂ | 35 | 24 |
| A. pleuropneumoniae type 2 | 14 | Pour | Sawada | CO₂ | 35 | 24 |
| H. parasuis | 15 | Pour | Sawada | CO₂ | 35 | 24 |
| P. multocida type D | 19 | Pour | MHA | Aerobic | 35 | 24 |
| P. haemolytica | 21 | Pour | MHA | Aerobic | 35 | 24 |
| P. trehalosi | ≤8 | Pour | MHA | Aerobic | 35 | 24 |
| E. coil | ≤8 | Pour | MHA | Aerobic | 35 | 24 |
| S. typhimurium | 10 | Pour | MHA | Aerobic | 35 | 24 |
| S. choleraesuis | 12 | Pour | MHA | Aerobic | 35 | 24 |
| K. pneumoniae ATCC 10031 | 10 | Pour | MHA | Aerobic | 35 | 24 |
| P. aeruginosa | ≤8 | Pour | MHA | Aerobic | 35 | 24 |
| P. aeruginosa pump-hyperexpressing strain | ≤8 | Pour | MHA | Aerobic | 35 | 24 |
| P. aeruginosa pump deficient strain | ≤8 | Pour | MHA | Aerobic | 35 | 24 |
| A. junii | 18 | Pour | MHA | Aerobic | 35 | 24 |
| Acinetobacter. sp. | 9 | Streak | MHA | Aerobic | 35 | 24 |
| S. maltophilia | 26 | Pour | MHA | Aerobic | 35 | 24 |
| L. pneumophila serotype 1 | 40 | Streak | B-CYE α | Aerobic | 35 | 48 |
| M. gallisepticum | 23 | Streak 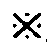 2 | Fray | CO₂ | 35 | 6 days |
| M. synoviae | 23 | Strek 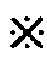 2 | Fray | CO₂ | 35 | 6 days |

**[Table 2-2]**

| Strain name | Inhibitory zone diameter (mm) | Assay method | Agar medium 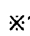 1 | Culture method | Culture temperature (°C) | Culture time (h) |
|---|---|---|---|---|---|---|
| C. jejuni | 25 | Streak | GAM | Microaerobic | 35 | 24 |
| C. coli | 26 | Streak | GAM | Microaerobic | 35 | 24 |
| H. pylori ATCC 43526 | 19 | Streak | MHA supplemented with sheep blood | Microaerobic | 35 | 4 days |
| C. perfringens | 18 | Pour | GAM | Anaerobic | 35 | 24 |
| C. difficile | 27 | Streak | GAM | Anaerobic | 35 | 24 |
| P. acnes JCM 6425 | 33 | Pour | GAM | Anaerobic | 35 | 48 |
| B. fragilis | 20 | Pour | GAM | Anaerobic | 35 | 24 |
| P. gingivalis JCM 8525 | 33 | Pour | GAM | Anaerobic | 35 | 48 |
| T. forsythensis JCM 10827 | 9 | Streak 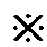 3 | MHA supplemented with sheep blood | Anaerobic | 35 | 5 days |
| L. acidophilus JCM 1132 | 13 | Pour | GAM | Anaerobic | 35 | 24 |
| B. bifidum JCM 1255 | 23 | Pour | GAM | Anaerobic | 35 | 48 |
| A. prevotii JCM 6508 | 21 | Pour | GAM | Anaerobic | 35 | 24 |
| F. nucleatum | 20 | Streak | GAM | Anaerobic | 35 | 24 |
| C. albicans | ≤8 | Streak | GAM | Aerobic | 35 | 24 |
| Cryptococcus sp. | ≤8 | Streak | GAM | Aerobic | 35 | 48 |
| A. niger | ≤8 | Streak 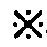 4 | PDA | Aerobic | 25 | 48 |
| F. oxysporum | 19 | Strek 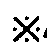 4 | PDA | Aerobic | 25 | 48 |

| | | | | | | |
|---|---|---|---|---|---|---|
| 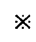 1: Agar medium MHA: Mueller-Hintons agar medium (manufactured by Eiken Chemical Co., Ltd.) GAM: GAM agar medium (manufactured by Nissui Pharmaceutical Co., Ltd.) Sawada: Sawada's medium (internally prepared) B-CYEα: B-CYEα agar medium (manufactured by Eiken Chemical Co. , Ltd.) Fray: Fray's medium (internally prepared) PDA: Potato dextrose agar medium (manufactured by Eiken Chemical Co., Ltd.) 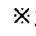 2: Streak method for M. gallisepticum and M. synoviae M. gallisepticum or M. synoviae was subcultured for three passages at 35°C using Fray' s broth (internally prepared) while the growth thereof was confirmed by the change of the color into yellow. A sterile cotton swab on a wood stick was impregnated with the broth of the third passage whose color had changed into yellow, and streaking was carried out in three directions on Fray's agar medium. 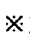 3: Streak method for T. forsythensis JCM 10827 T. for sythensis requires N-acetylmuramic acid for its growth, therefore, it was cultured by placing a disk containing 1.5% N-acetylmuramic acid on the center of the medium. Fifteen milligrams of N-acetylmuramic acid was dissolved in 1 ml of purified water, and the solution was filtered with a 0.45 µm filter. Then, 20 µl of the solution was impregnated in respective paper disks with a diameter of 8 mm, and the disks were dried at room temperature, which were used as disks containing 1.5% N-acetylmuramic acid. 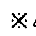 4: Streak method for A. niger and F. oxysporum A. niger and F. oxysporum were cultured at 25°C for 7 days using a PDA slant medium. To the slant medium on which the fungi were grown, 2 ml of physiological saline supplemented with 0.1% Tween 80 was added and mixed gently, whereby a spore solution was prepared. The spore solution was put in a hemocytometer and the number of spores was determined. Then, the spore number was adjusted to 1 x 10⁶/ml with physiological saline supplemented with 0.1% Tween 80. A sterile cotton swab on a wood stick was impregnated with the spore solution, and streaking was carried out in three directions on a PDA plate. | | | | | | |

The above results demonstrated that DM0507 of the present invention has an extremely broad antimicrobial spectrum, and the potency of the antimicrobial activity varies depending on the strains.

### Example 3

### Production of astringent lotion

An astringent lotion of the following formulation was produced by mixing the respective components based on a standard method.

| (Formulation) | (Compounding amount: % by mass) |
|---|---|
| Glycerin | 3.0 |
| Sorbitol | 2.0 |
| Hydrophilic surfactant | 1.0 |
| Ethanol | 14.0 |
| Citrate/phosphate buffer | 0.1 |
| Zinc paraphenolsulfonate | 0.2 |
| DM0507* | 1.0 |
| Flavor | q.s. |
| Preservative | q.s. |
| Purified water | 78.7 |

| | |
|---|---|
| * produced in Example 1 | |

### Example 4

### Production of emulsion

An emulsion of the following formulation was produced by mixing the respective components based on a standard method.

| (Formulation) | (Compounding amount: % by mass) |
|---|---|
| Bees wax | 0.5 |
| Vaseline | 2.0 |
| Squalan | 5.0 |
| Lipophilic emulsifying agent | 0.8 |
| Hydrophilic emulsifying agent | 1.2 |
| Flavor | 0.5 |
| Preservative | q.s. |
| Antioxidant | q.s. |
| Propyleneglycol | 5.0 |
| Ethanol | 4.0 |
| Aqueous solution of thickening agent (1%) | 20.0 |
| Potassium hydroxide | 0.1 |
| DM0507* | 1.0 |
| Purified water | 59.9 |

| | |
|---|---|
| * produced in Example 1 | |

### Example 5

### Production of therapeutic dentifrice

A therapeutic dentifrice of the following formulation was produced by mixing the respective components based on a standard method.

| (Formulation) | (Compounding amount: % by mass) |
|---|---|
| Cetylpyridinium chloride | 0.01 |
| Sodium chloride | 10.0 |
| Calcium hydrogen phosphate | 20.0 |
| Tragacanth | 2.0 |
| Sodium citrate | q.s. |
| Citric acid | q.s. |
| p-hydroxybenzoate ester | 0.5 |
| Purified water | Balance |
| Flavor | a very small amount |
| Legal dye | a very small amount |
| DM0507* | 1.0 |

| | |
|---|---|
| * produced in Example 1 | |

### Example 6

### Production of medical soap

A medical soap of the following formulation was produced by mixing the respective components based on a standard method.

| (Formulation) | (Compounding amount: % by mass) |
|---|---|
| Triclosan | 0.3 |
| Polyoxyethylene | q.s. |
| Lauryl ether phosphate | 2.0 |
| Mixed plant extract | 2.0 |
| Substrate for soap | 35.0 |
| Citric acid | q.s. |
| p-hydroxybenzoate ester | 0.5 |
| Purified water | Balance |
| Flavor | a very small amount |
| Legal dye | a very small amount |
| DM0507* | 1.0 |

| | |
|---|---|
| * produced in Example 1 | |

### Example 7

### Production of beautifying essence

A beautifying essence of the following formulation was produced by mixing the respective components based on a standard method.

| (Formulation) | (Compounding amount: % by mass |
|---|---|
| Glycerin | 5.0 |
| BG | 10.0 |
| Hyaluronic acid | 0.01 |
| Collagen | 0.8 |
| Amino acid | 0.5 |
| Plant extract | 0.5 |
| Carbomer | 0.2 |
| Paraben | 0.2 |
| Purified water | Balance |
| DM0507* | 1.0 |

| | |
|---|---|
| * produced in Example 1 | |

### Example 8

### Production of cream

A cream of the following formulation was produced by mixing the respective components based on a standard method.

| (Formulation) | (Compounding amount: % by mass) |
|---|---|
| Glycerin | 6.0 |
| BG | 7.0 |
| Hyaluronic acid | 0.01 |
| Plant extract | 0.5 |
| Squalan | 10.0 |
| Stearic acid | 2.0 |
| Behenyl alcohol | 5.0 |
| Paraben | 0.2 |
| Purified water | Balance |
| DM0507* | 1.0 |

| | |
|---|---|
| * produced in Example 1 | |

### Example 9

### Production of cleansing agent

A cleansing agent of the following formulation was produced by mixing the respective components based on a standard method.

| (Formulation) | (Compounding amount: % by mass) |
|---|---|
| Glycerin | 3.0 |
| BG | 3.0 |
| Stearic acid | 5.2 |
| Mineral oil | 15.0 |
| Dialkyl carbonate | 10.0 |
| Cocoyl methyl taurate | 0.5 |
| Cetearyl alcohol | 1.5 |
| Paraben | 0.2 |
| Purified water | Balance |
| DM0507* | 1.0 |

| | |
|---|---|
| * produced in Example 1 | |

### Example 10

### Production of bath agent

A bath agent of the following formulation was produced by mixing the respective components based on a standard method.

| (Formulation) | (Compounding amount: % by mass) |
|---|---|
| Sodium hydrogen carbonate | 50.0 |
| Dry sodium sulfide | 30.0 |
| Titanium chloride | 1.2 |
| Legal dye | a very small amount |
| Flavor | a very small amount |
| Polyethyleneglycol | Balance |
| DM0507* | 0.001 |

| | |
|---|---|
| * produced in Example 1 and then powderized | |

### Industrial Applicability

Since DM0507 of the present invention has an antimicrobial activity against a broad spectrum of strains, it can be used for an antimicrobial agent, antimicrobial product, a food or drink, etc. having an excellent antimicrobial activity.

### Brief Description of the Drawings

[Fig. 1] It is a chart showing the IR spectrum of DM0507.
[Fig. 2] It is a chart showing the NMR spectrum of DM0507.

## Claims

1. An antimicrobial active substance DM0507, **characterized by** obtaining by a production process comprising the following steps (a) to (d):
(a) culturing Bacillus subtilis;
(b) collecting the supernatant from the obtained culture;
(c) collecting the precipitate formed by adjusting the pH of the supernatant to 3 or lower; and
(d) performing extraction from the precipitate with ethanol.

2. The antimicrobial active substance DM0507 according to Claim 1, having an antimicrobial activity against the following microorganism Groups (A) to (D):
Group (A) (aerobic/ facultative anaerobic microorganisms)
Streptococcus suis,
Streptococcus pneumoniae,
Streptococcus pyogenes,
Bacillus subtilis ATCC 6633,
Bacillus cereus var. mycoides ATCC 11778,
Arcanobacterium pyogenes,
Aeromonas salmonicida,
Aeromonas hydrophila,
Actinobacillus actinomycetemcomitans JCM 2434, Actinobacillus pleuropneumoniae type 2,
Haemophilus parasuis,
Pasteurella multocida type D,
Pasteurella haemolytica,
Salmonella typhimurium,
Salmonella choleraesuis,
Klebsiella pneumoniae ATCC 10031,
Acinetobacter junii,
Acinetobacter sp.,
Stenotrophomonas maltophilia,
Legionella pneumophila serotype 1,
Mycoplasma gallisepticum, and
Mycoplasma synoviae;
Group (B) (microaerophilic microorganisms)
Campylobacter jejuni,
Campylobacter coli, and
Helicobacter pylori ATCC 43526;
Group (C) (obligate anaerobic microorganisms)
Clostridium perfringens,
Clostridium difficile,
Propionibacterium acnes JCM 6425,
Bacteroides fragilis,
Porphyromonas gingivalis JCM 8525,
Tannerella forsythensis JCM 10827,
Lactobacillus acidophilus JCM 1132,
Bifidobacterium bifidum JCM 1255,
Anaerococcus prevotii JCM 6508, and
Fusobacterium nucleatum; and
Group (D) (fungi)
Fusarium oxysporum.

3. The antimicrobial active substance DM0507 according to Claim 1 or 2, wherein the Bacillus subtilis is Bacillus subtilis DB9011 (FERM BP-3418).

4. An antimicrobial agent containing an antimicrobial active substance DM0507 according to any one of Claims 1 to 3 as an active ingredient.

5. A food or drink containing an antimicrobial active substance DM0507 according to any one of Claims 1 to 3.

6. A process for producing an antimicrobial active substance DM0507, **characterized by** comprising the following steps (a) to (d):
(a) culturing Bacillus subtilis;
(b) collecting the supernatant from the obtained culture;
(c) collecting the precipitate formed by adjusting the pH of the supernatant to 3 or lower; and
(d) performing extraction from the precipitate with ethanol.
